# EUROPEAN PATENT SPECIFICATION

(11) **EP 3 323 801 B1**
(45) Date of publication and mention of the grant of the patent: **24.06.2020**
(21) Application number: 16382539.1
(22) Date of filing: 18.11.2016
(51) Int. Cl.: C07C 45/00

(54) **METHODS OF PREPARING CYCLOHEXANONE AND DERIVATIVES**
VERFAHREN ZUR REPARATUR VON CYCLOHEXANON UND DERIVATEN
PROCÉDÉS DE PRÉPARATION DE CYCLOHEXANONE ET DE DÉRIVÉS

(43) Date of publication of application: 23.05.2018
(73) Proprietor: International Flavors & Fragrances Inc., New York, NY 10019 (US)
(72) Inventor: GONELL GÓMEZ, Francisco, 46010 Valencia (ES); IBORRA CHORNET, Sara, 46006 Valencia (ES); CORMA CANÓS, Avelino, 46020 Valencia (ES); ESPINOS FERRI, Estela, 12580 Moncofa Castellón (ES)
(74) Representative: ABG Intellectual Property Law, S.L.

(56) References cited:
- DE-A1- 2 059 938
- DE-A1- 2 909 780
- GB-A- 1 063 357

## Description

### FIELD OF THE INVENTION

The present invention relates to new hydrogenation methods on substituted phenols for the manufacturing of cyclohexanones, in particular, production of 4-*tert-*amylcyclohexanone ("Orivone") by hydrogenation of 4-*tert*-amylphenol in good selectivity and high yields.

### BACKGROUND OF THE INVENTION

Cyclohexanone and its derivatives have been employed in various industrial processes and products. Cyclohexanone, for example, can be used as a solvent or an activator in oxidation reactions. It can also be used as an intermediate, for example, in the production of resins, caprolactam, adipic acid or nylon-6,6. Orivone (*i.e.,* 4-tert-amylcyclohexanone), a derivative of cyclohexanone, is a fragrance compound widely used in the manufacturing of perfume compositions or products, including soaps, detergents, cosmetic powders, and liquid detergents.

Cyclohexanone and its derivatives such as Orivone have been prepared by catalytic hydrogenation of phenol or substituted phenols in a hydrogenation reactor. See, e.g., Cerveny et al., Journal of Molecular Catalysis A: Chemical 2003, 194, 249-254; Goettmann et al., Chemical Communications, 2008, 999-1001; Wydra et al., Chemie Ingenieur Technik, 2002, 74, 800; Corma et al., Applied Catalysis A: General 2011, 404, 103-112; Catalysis Communications 2011, 12, 1071-1075); KR2009118321; DE2909780 ; and U.S. Patent No. 8772550. Patent application GB1063357 is related to a process for the synthesis of cyclohexanone by selective hydrogenation of phenol by means of a catalyst containing palladium at high temperatures, with the active species supported on alumina. The invention disclosed in patent application DE2059938 refers to the process for the preparation of cyclohexanone by catalytic hydrogenation of phenol at high temperatures using metal species of the platinum group supported on alumina or silica.

However, a limitation of the conventional hydrogenation processes is the moderate selectivity of cyclohexanone over cyclohexanol, among others. In addition, the process often requires a solvent disposal step, which may cause environmental concerns. Further, some processes involve steam for heating and result in increased energy consumption.

Therefore, an efficient and effective method for hydrogenation of substituted phenols with an improved selectivity for the production of cyclohexanones is highly desirable.

### SUMMARY OF THE INVENTION

The scope of the present invention is as is defined by the appended claims. The method of the present invention generally comprises the steps of reducing an optionally substituted phenol (in other words, a substituted or unsubstituted phenol) in the presence of a catalyst using hydrogen in a reactor substantially free of a solvent, thereby obtaining an optionally substituted cyclohexanone. The catalyst includes a catalytically active transition metal, a support containing an oxide (*e.g.,* a metal oxide), and a promoter containing an inorganic metal salt.

In some embodiments, the catalytically active transition metal is selected from the group consisting of Rh, Ru, Ir, Ni, Pd, Au, and Pt. A preferred catalytically active transition metal is Pd. In some embodiments, the oxide is a metal oxide containing at least one oxygen atom and an element selected from the group consisting of an alkaline-earth metal, a transition metal, a lanthanide, and a p-block element metal. Preferred oxides include SiO₂, MgO, Al₂O₃, TiO₂, CeO₂, and ZrO₂. In some embodiments, the promoter is an alkaline metal salt, an alkaline-earth metal salt, or a combination thereof. Preferred alkaline metals and alkaline-earth metals include Li, Na, K, Rb, Cs, Be, Mg, Ca, Sr, and Ba.

The catalytically active transition metal is generally present in an amount of between about 0.1% and about 10% (*e.g.,* 0.5% and 6%) by weight of the catalyst. The promoter is typically present in an amount of between about 0.01% and about 10% (*e.g.,* 0.05% and 5%) by weight of the catalyst.

The catalyst may be a reduced catalyst, which is, prior to the reduction reaction, treated with a hydrogen gas at a flow rate of about 50 mL/min to about 150 mL/min and a temperature ranging from about 100 °C to about 400 °C. The reduction reaction can be carried out in liquid phase or gas phase, in a batch system or in a continuous reactor, and/or at a temperature ranging between about 80 °C and about 250 °C. The catalyst is generally in an amount of between about 0.5% and about 10% (*e.g.,* about 1% and about 5%) by weight of the substituted or unsubstituted phenol.

The phenol is optionally substituted with 1, 2, 3, 4, or 5 substituents. In some embodiments, each of the substituents is, independently, a C₁-C₁₂ (*e.g.,* C₁-C₈) alkyl group. In some embodiments, each of the substituents is independently selected from the group consisting of methyl, ethyl, propyl, isopropyl, tert-butyl, isobutyl, tert-amyl, isoamyl, n-hexyl, 1-methylpentyl and 1,1-dimethylbutyl. In some embodiment, the optionally substituted phenol is 4-tert-amylphenol and the optionally substituted cyclohexanone is 4-tert-amylcyclohexanone (Orivone).

Additional advantages of the method will be realized and attained by the methods and systems described and illustrated in the written description and claims hereof.

### DETAILED DESCRIPTION OF THE INVENTION

Various embodiments of the present disclosure provide a novel approach to reduction of substituted or unsubstituted phenols via hydrogenation. Advantages of this approach over the conventional processes include high selectivity for desired cyclohexanones, minimum generation of waste, and low cost.

Throughout this patent document, various publications or patents are referenced. The disclosures of these publications or patents in their entireties are hereby incorporated by reference into this application in order to more fully describe the state of the art to which the disclosed matter pertains. While the following text may reference or exemplify specific components of a device or a method of utilizing the device, it is not intended to limit the scope of the invention to such particular references or examples. Various modifications may be made by those skilled in the art, in view of practical and economic considerations, such as the amount of the catalyst and the pressure of the hydrogenation condition. In order to more clearly and concisely describe the subject matter of the claims, the following definitions are intended to provide guidance as to the meaning of terms used herein.

The articles "a" and "an" as used herein refers to "one or more" or "at least one," unless otherwise indicated. That is, reference to any element or component of the present disclosure by the indefinite article "a" or "an" does not exclude the possibility that more than one element or component is present.

The term "about" as used herein refers to the referenced numeric indication plus or minus 10%, preferably within plus or minus 5%, of that referenced numeric indication.

The term "phenols" in plural form as used herein refers to unsubstituted and substituted phenol. Similarly, the term "cyclohexanones" refers to unsubstituted and substituted cyclohexanone.

The term "C₁-C₁₂" includes alkyls containing 12, 11, 10, 9, 8, 7, 6, 5, 4, 3, 2, or 1 carbon atoms. Similarly, "C₁-C₈" includes alkyls containing 1 to 8 carbons.

The term "alkoxy" as used herein, alone or in combination, includes an alkyl group connected to the oxy connecting atom. The term "alkoxy" also includes alkyl ether groups, where the term 'alkyl' is defined above, and 'ether' means two alkyl groups with an oxygen atom between them. Examples of suitable alkoxy groups include methoxy, ethoxy, n-propoxy, i-propoxy, n-butoxy, s-butoxy, t-butoxy, methoxymethane, and methoxyethane.

The term "solvent" as used herein includes all organic solvents, water and any mixture thereof.

Although various hydrogenation processes are known in the art, the conversion of phenols and the selectivity for desired cyclohexanones are often at issue in conventional manufacturing approaches. Meanwhile, use of solvents adds to the energy cost and is environmentally unfriendly.

An aspect of the invention provides a method of preparing a substituted or unsubstituted cyclohexanone by reducing a substituted or unsubstituted phenol via a hydrogenation reaction. The method generally includes contacting a substituted or unsubstituted phenol with a catalyst in the presence of a hydrogen gas. The reduction reaction takes place in a condition substantially free of an additional solvent other than the substituted or unsubstituted phenol itself. As a result, high conversion of the substituted or unsubstituted phenol is achieved. Moreover, the amount of side products, *e.g.,* the corresponding cyclohexanols, is significantly reduced.

The phenol substrate of the hydrogenation reaction is an unsubstituted phenol or a substituted phenol having one or more substituents. Suitable substituents include C₁-C₁₀ alkyl, alkoxy, amino, ether, amide, and ester. The substituents can be located at one or more of ortho, meta, and para positions of the hydroxyl moiety of phenol. In some embodiments, the substituted phenol has one or more alkyl groups selected from the group consisting of methyl, ethyl, *n*-propyl, *iso*-propyl, *n*-butyl, *iso-*butyl, *tert*-butyl, 1,1-dimethylpropyl (namely, *tert*-amyl), 1-methylbutyl, and n-pentyl. In some embodiments, the substituted phenol is 4-*tert*-amyl substituted phenol and the hydrogenation product is Orivone.

Optionally substituted cyclohexanones and phenols can be illustrated in Formulas (I) and (II) as follows: 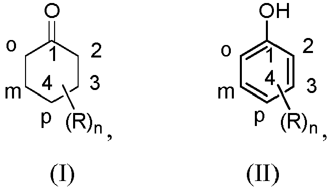 wherein:
n is 0, 1, 2, 3, 4, or 5; and
R in each occurrence is independently a C₁-C₁₂ alkyl group at any of the two ortho (o), two meta (m), and one para (p) positions. Using the numbers 1, 2, 3, and 4 are another way to point out the substitution position on the phenol or cyclohexanone ring.

In a particular embodiment, the substituted cyclohexanone is Orivone (4-tert-amylcyclohexanone) having the structure of Formula (III) and the substituted phenol is 4-*tert*-amylphenol having the structure of Formula (IV) as follows:

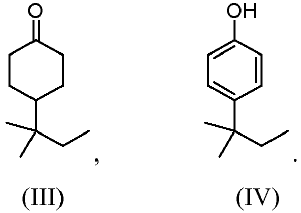

The catalyst for the hydrogenation reaction generally contains a catalytically active transition metal, a support and a promoter. Additional components facilitating the reaction may also be included in the catalyst.

The catalytically active transition metal may be selected from the group consisting of rhodium (Rh), ruthenium (Ru), iridium (Ir), nickel (Ni), palladium (Pd), platinum (Pt), gold (Au), rubidium (Rb), osmium (Os), and any combinations of these metals. In some embodiments, the catalytically active transition metal is Pd. The amount of the catalytically active transition metal in the catalyst may vary depending on specific metal, substrate, reaction condition and other factors. Non-limiting examples of the range (by weight in the catalyst) of the catalytically active transition metal include about 0.1% to about 15%, about 0.1% to about 10%, about 0.2% to about 10%, about 0.5% to about 10%, about 0.8% to about 10%, about 1% to about 10%, about 0.1% to about 8%, about 0.1% to about 6%, about 0.1% to about 2%, about 0.1% to about 1%, about 0.5% to about 10%, about 0.5% to about 6%, about 0.5% to about 2%, and about 0.5% to about 1%, all subranges and subvalues included. Further examples of the amount of the catalytically active metal include about 0.1%, about 0.5%, about 1%, about 3%, about 5%, about 6%, about 7%, and about 10%, all by weight of the catalyst.

The catalytically active transition metals can be used in the form of a salt or as a reduced metal. Exemplary palladium salts are palladium(II) acetylacetonate (Pd(acac)₂), palladium(II) chloride (PdCl₂), and bis(benzonitrile)palladium(II) chloride (PdCl₂(PhCN)₂).

The catalytically active metal can be deposited on a support by impregnation or precipitation. The support may include one or more materials such as alumina, activated carbon, titanium oxide, calcium carbonate and carbon black. Another support that may be used is silica. In some embodiments, the support contains a metal oxide, wherein the metal contained therein can be an alkaline-earth metal, a transition metal, a lanthanide, or a p-block element metal. One or more metal oxides can be included in the catalyst. Non-limiting examples include SiO₂, MgO, Al₂O₃, TiO₂, CeO₂, and ZrO₂. In some embodiments, the support contains or consists of Al₂O₃.

The amount of the support may vary from about 70% to about 99.9% by weight of the catalyst. Exemplary ranges include from about 80% to about 99.8%, from about 85% to about 99.8%, from about 90% to about 99.8%, from about 95% to about 99.8%, and from about 98% to about 99.8%, all subranges and subvalues included.

The catalyst further contains a promoter comprising one or more salts of an alkali or alkaline-earth metal. In some embodiments, the promoter is a salt of a metal. The metal can be selected from the group consisting of Li, Na, K, Rb, Cs, Be, Mg, Ca, Sr, Ba, and combinations thereof. In some embodiments, the anion of the salt is selected from the group consisting of hydroxide, carbonate, bicarbonate, sulfate, bisulfate, halide (fluoride, chloride, bromide, and iodide), nitrate, and combinations thereof. In some embodiments, the salt is selected from the group consisting of sodium carbonate, potassium carbonate, calcium carbonate, magnesium carbonate, and combinations thereof. The promoter is generally introduced to the support by impregnation.

The amount of the promoter in the catalyst depends on various factors, including the specific substrate, catalytically active metal, support, and reaction conditions. Non-limiting examples of the range (by weight of the catalyst) of the promoter include about 0.1% to about 15%, about 0.1% to about 10%, about 0.2% to about 10%, about 0.5% to about 10%, about 0.8% to about 10%, about 1% to about 10%, about 0.1% to about 8%, about 0.1% to about 5%, about 0.1% to about 2%, about 0.1% to about 1%, about 0.5% to about 10%, about 0.5% to about 5%, about 0.5% to about 2.5%, about 0.5% to about 2%, and about 0.5% to about 1%, all subranges and subvalues included. Further examples include about 0.1%, about 0.5%, about 1%, about 3%, about 5%, about 8%, and about 10%, all by weight of the catalyst.

The amount of the catalyst by weight of the substituted or unsubstituted phenol may range from about 0.1% to about 15%, from about 0.1% to about 10%, from about 0.5% to about 15%, from about 0.5% to about 10%, from about 1% to about 10%, or from about 1% to about 5%,. Additional examples include about 0.2%, about 0.5%, about 0.8%, about 1%, about 2%, about 3%, about 5%, about 8%, and about 10%.

The catalyst may be a reduced catalyst, which is treated with a hydrogen gas prior to the hydrogenation reaction of the phenol. In exemplary embodiments, the catalyst undergoes treatment in a hydrogen atmosphere. Heating may be applied to reach a temperature of from about 50 °C to about 500 °C for 1 minute to 24 hours (*e.g.,* 10 minutes to 16 hours, 30 minutes to 12 hours, 1 to 10 hours, and 1 to 5 hours). The hydrogen pressure may be set at a range of from about 1 to about 20 bars.

In some embodiments, the catalyst is calcined in the presence of air prior to the hydrogenation of phenols. The calcining temperature ranges from about 100 to about 600 °C, from about 150 to about 500 °C, or from about 250 to about 500 °C. The catalyst can then be reduced under a hydrogen flow at a rate ranging from about 20 to about 200 ml/min, from about 40 to about 160, from about 50 to about 150, or from about 50 to about 100 ml/min. The temperature can be controlled at a range of from about 100 to about 500, or from about 100 to about 400 °C.

The yield and selectivity of the method do not rely on the presence of an organic solvent or water. It is preferred that no organic solvent or water is added before and/or during the hydrogenation reaction. In some embodiments, the reaction condition is substantially free from an organic solvent, water, or any combination thereof. In some embodiments, the amount of organic solvent or water in the reaction system is less than about 5% (*e.g.,* 0-5%), less than about 4% (0-4%), less than about 3% (0-3%), less than about 2% (0-2%), less than about 1% (0-1%), less than about 0.8% (0-0.8%), less than about 0.5% (0-0.5%), less than about 0.2% (0-0.2%), or less than about 0.1% (0-0.1%) by weight. The reaction may be carried out in liquid phase or gas phase, and/or in a batch system or in a continuous reactor.

The hydrogen for reducing the phenols may have been pressurized ranging from about 0.5 to about 25 bars. The reactor in many cases needs to be purged prior to the reaction so that the gas phase is substantially free from oxygen. Non-limiting examples of the hydrogen pressure range include from about 1 to about 15 bars, from about 1 to about 10 bars, from about 1 to about 8 bars, from about 1 to about 5 bars, from about 5 to about 10 bars, and from about 10 to about 15 bars, all subranges and subvalues included. Further exemplary hydrogen pressures include about 1, about 2, about 3, about 5 about 8, about 10, about 12, and about 15 bars.

In some embodiments, the hydrogenation process takes place under a low flow rate of hydrogen gas. The flow rate typically ranges from about 0.1 to about 150 mL/min, from about 0.1 to about 100 mL/min, from about 0.1 to about 50 mL/min, from about 1 to about 100 mL/min, from about 1 to about 50 mL/min, from about 10 to about 100 mL/min, from about 0.2 to about 50 mL/min, from about 0.2 to about 20 mL/min, from about 0.5 to about 20 mL/min, or from about 0.5 to about 10 mL/min.

The temperature of the hydrogenation process may range from about room temperature (*i.e.,* 25 °C) to about 350 °C. Exemplary temperature ranges include from about 50 °C to about 300 °C, from about 50 °C to about 250 °C, from about 50 °C to about 200 °C, from about 50 °C to about 150 °C, from about 50 °C to about 100 °C, from about 80 °C to about 250 °C, from about 80 °C to about 200 °C, from about 80 °C to about 170 °C, from about 90 °C to about 250 °C, from about 90 °C to about 170 °C, from about 90 °C to about 150 °C, and from about 100 °C to about 150 °C, all subranges and subvalues included.

The reaction time may vary depending on the substrate, the catalyst and other factors. Exemplary ranges of the reaction time include from about 1 to about 20 hours, from about 5 to about 15 hours, from about 8 to about 12 hours, and from about 10 to about 15 hours, all subranges and subvalues included. Further examples of reaction time include about 5, about 8, about 10, about 12, and about 15 hours.

The hydrogenation reaction may take place in any suitable reactor such as a batch semi-continuous or continuous reactor. Examples of reactors include packed bed reactors, slurry reactors, shell and tubes heat exchange reactors. The substrate and the catalyst may be shaken, stirred, spun to ensure thorough mixing.

The method of the present disclosure is able to achieve high conversion rate and selectivity. The conversion rates of the phenols include, for example, more than about 70% (*e.g.,* 70-100%), more than about 75% (75-100%), more than about 80% (80-100%), more than about 85% (85-100%), more than about 90% (90-100%), and more than about 95% (95-100%). The conversion rate is calculated as mass of the phenol consumed/total mass of the phenol fed into the reaction x 100%.

High levels of selectivity for cyclohexanones can also be achieved. Examples of the selectivity for cyclohexanones over the corresponding cyclohexanols include more than about 80% (*e.g.,* 80-100%), more than about 85%, more than about 88%, more than about 90%, more than about 92%, more than about 94%, more than about 96%, and more than about 98%.

The following non-limiting examples further illustrate certain aspects of the present invention.

### EXAMPLES

### EXAMPLE 1. Catalyst preparation

Preparation of Pd-γ-Al₂O₃: 0.143 g of palladium acetylacetonate (Pd(acac)₂) was dissolved in 25 mL of acetone, this solution was impregnated, at 40°C, to 1 g of y-Al₂O₃. The resulting Pd- γ-Al₂O₃ contains 5% by weight of Pd. After impregnation the catalyst was dried at 100°C for 2 hours, and calcined at 450°C for 2 hours at a heating rate of 3.5°C/min in a muffle furnace.
CATALYST 1: 5 mL of an aqueous solution of 5.8 mg of Na₂CO₃ was introduced to 0.5 g of catalyst Pd-γ-Al₂O₃ prepared above by impregnation at 80°C. After drying the catalyst at 100°C for 2 hours, it was reduced at 150°C for 2 hours under H₂ flow to obtain Catalyst 1 having 0.5% Na by weight.
CATALYST 2: 5 mL of an aqueous solution of 11.6 mg of Na₂CO₃ was introduced to 0.5 g of catalyst Pd-γ-Al₂O₃ prepared above by impregnation at 80°C. After drying the catalyst at 100°C for 2 hours, it was reduced at 150°C for 2 hours under H₂ flow to obtain Catalyst 2 having 1% Na by weight.
CATALYST 3: 5 mL of an aqueous solution of 17.4 mg of Na₂CO₃ was introduced to 0.5 g of Pd-γ-Al₂O₃ by impregnation at 80°C. After drying the catalyst at 100°C for 2 hours, it was reduced at 150°C for 2 hours under H₂ flow to obtain Catalyst 3 having 1.5% Na by weight.
CATALYST 4: In this case 5 mL of an aqueous solution of 23.1 mg of Na₂CO₃ was introduced to 0.5 g of Pd-γ-Al₂O₃ by impregnation at 80°C. After drying the catalyst at 100°C for 2 hours, it was reduced at 150°C for 2 hours under H₂ flow to obtain Catalyst 4 having 2% Na by weight.
CATALYST 5: In this case 5 mL of an aqueous solution of 28.9 mg of Na₂CO₃ was introduced to 0.5 g of Pd-γ-Al₂O₃ by impregnation at 80°C. After drying the catalyst at 100°C for 2 hours, it was reduced at 150°C for 2 hours under H₂ flow to obtain Catalyst 5 having 2.5% Na by weight.
CATALYST 6: In this case 5 mL of an aqueous solution of 57.9 mg of Na₂CO₃ was introduced to 0.5 g of Pd-γ-Al₂O₃ by impregnation at 80°C. After drying the catalyst at 100°C for 2 hours, it was reduced at 150°C for 2 hours under H₂ flow to obtain Catalyst 6 having 5% Na by weight.

### EXAMPLE 2. p-tert-amylphenol hydrogenation with Catalysts 1-6

Hydrogenation reactions were carried out in a reinforced glass reactor equipped with a manometer to monitor the pressure in the reactor. To the reactor were added 10.5 mg of one of the catalysts and 495 mg of *p-tert*-amylphenol. The reactor was then purged with hydrogen, pressurized at 5 bars (dynamic pressure) with hydrogen, heated to 100°C, and kept at that temperature for a period of time (*e.g.,* 10 to 16 hours). The quantitative analysis of the reaction mixture after the filtration was carried out in a GC-MS and GC-FID. Conversion was calculated as the moles of *p-tert*-amylphenol converted/the initial moles of this compound x 100%. The yield of the desired product Orivone or byproduct *4-tert*-amylcyclohexanol was determined as the moles of the compound/the initial moles of *p-tert*-amylphenol x 100%. Orivone selectivity was calculated as the moles of Orivone/the moles of *p-tert*-amylphenol converted x 100%.

Table 1 below summarizes the results obtained in the hydrogenation reactions with Catalysts 1-6. The reaction time was 10 hours. In this table, "Conversion" refers to the conversion rate of *p-tert*-amylphenol, "OL Yield" refers to the yield of 4-*tert*-amylcyclohexanol, "ONE Yield" refers to the yield of Orivone, and "ONE Selectivity" refers to the selectivity of Orivone.

**Table 1**

| Catalyst | Conversion (%) | OL Yield (%) | ONE Yield (%) | ONE Selectivity (%) |
|---|---|---|---|---|
| Pd-γ-Al₂O₃ | 61.6 | 6.1 | 55.4 | 90.1 |
| Catalyst 1 | 87.2 | 6.6 | 80.6 | 92.4 |
| Catalyst 2 | 95.1 | 11.6 | 83.5 | 87.8 |
| Catalyst 3 | 87.9 | 6.3 | 81.6 | 92.9 |
| Catalyst 4 | 85.0 | 7.8 | 77.2 | 90.8 |
| Catalyst 5 | 77.3 | 5.9 | 71.3 | 92.4 |
| Catalyst 6 | 58.4 | 2.3 | 56.1 | 96.1 |

### EXAMPLE 3. p-tert-amylphenol hydrogenation with Catalyst 2 (5% Pd and 1% Na on γ-Al₂O₃) with or without a solvent

Hydrogenation reactions were carried out in a reinforced glass reactor equipped with a manometer. 10.5 mg of the catalyst, 495 mg of p-tert-amylphenol and 500 mg of solvent (water or tetrahydrofuran (THF)) were introduced to the reactor which was purged with hydrogen and pressurized at 5 bars (dynamic pressure) of hydrogen, and finally the reactor was heated at 100°C. The quantitative analysis of the reaction mixture after the filtration was carried out in a GC-MS and GC-FID. Table 2 below lists the results obtained with the Catalyst 2, performed in the absence of solvent and compared with THF and water are used as solvents.

**TABLE 2**

| **Solvent** | **Time (h)** | **Conversion (%)** | **OL Yield (%)** | **ONA Yield (%)** |
|---|---|---|---|---|
| No solvent | 10 | 95.1 | 11.6 | 83.5 |
| THF | 24 | 2 | 0.4 | 1.6 |
| H₂O | 24 | 2 | 0.5 | 1.5 |

In the claims, the term "comprises/comprising" does not exclude the presence of other elements or steps. Furthermore, although individually listed, a plurality of means, elements or method steps may be implemented by, e.g., a single unit or processor. Additionally, although individual features may be included in different claims, these may possibly advantageously be combined, and the inclusion in different claims does not imply that a combination of features is not feasible and/or advantageous.

The present disclosure is directed to a person of ordinary skill in the art. The purpose and advantages of the hydrogenation method will be set forth in, and be apparent from, the description that follows, as well as will be learned by practicing embodiments made in accordance with the disclosure.

## Claims

1. A method of preparing an optionally substituted cyclohexanone, comprising reducing an optionally substituted phenol in the presence of a catalyst and hydrogen in a reactor substantially free of a solvent, thereby obtaining the optionally substituted cyclohexanone;
wherein the catalyst comprises (i) a catalytically active transition metal, (ii) a support comprising an oxide, and (iii) a promoter comprising an inorganic metal salt,
wherein the promoter is present in an amount of between 0.5 to 2.5% by weight of the catalyst; and
wherein the promoter comprises a metal salt selected from the group consisting of Li, Na, K, Rb, and Cs, and preferably the promoter comprises sodium carbonate, potassium carbonate, or a combination thereof.

2. The method of claim 1, wherein the catalytically active transition metal is Rh, Ru, Ir, Ni, Pd, Au, Pt, or a combination thereof, and preferably Pd.

3. The method of claim 1 or 2, wherein the oxide is derived from an element selected from the group consisting of an alkaline-earth metal, a transition metal, a lanthanide, and a p-block element metal.

4. The method of any one of the preceding claims, wherein the oxide is SiO₂, MgO, Al₂O₃, TiO₂, CeO₂, ZrO₂, or a combination thereof, and preferably the oxide comprises Al₂O₃.

5. The method of any one of the preceding claims, wherein the catalytically active transition metal is present in an amount of between 0.1% and 10% by weight of the catalyst, and preferably between 0.5% and 6% by weight of the catalyst.

6. The method of any one of the preceding claims, wherein the catalyst is a reduced catalyst that is treated with a hydrogen gas at a flow rate of 50 mL/min to 150 mL/min and at a temperature of 100 °C to 400 °C.

7. The method of any one of the preceding claims, wherein the catalyst contacts the optionally substituted phenol under a hydrogen pressure between 1 bar and 20 bars.

8. The method of any one of the preceding claims, wherein the hydrogen reacts with the optionally substituted phenol in liquid phase or gas phase, in a batch system or in a continuous reactor, or at a temperature of between 80 °C and 250 °C, and preferably between 90 °C and 170 °C.

9. The method of any one of the preceding claims, wherein the catalyst is present in an amount of between 0.5% and 10% by weight of the optionally substituted phenol, and preferably between 1% and 5%.

10. The method of any one of the preceding claims, wherein the optionally substituted phenol is substituted with 1 to 5 substituents, each of which is, independently, a C₁-C₁₂ alkyl group.

11. The method of any one of the preceding claims, wherein the optionally substituted phenol is substituted with a C₁-C₁₂ alkyl at the 4- position, and the C₁-C₁₂ alkyl is selected from the group consisting of methyl, ethyl, propyl, *iso*propyl, *tert*-butyl, *iso*butyl, *tert*-amyl, *iso*amyl, *n*-hexyl, 1-methylpentyl, and 1,1-dimethylbutyl.

12. The method of any one of the preceding claims, wherein the optionally substituted cyclohexanone is 4-*tert*-amylcyclohexanone, and the optionally substituted phenol is 4-*tert*-amylphenol.

13. The method of any one of the preceding claims, wherein the catalyst comprises Pd as the catalytically active transition metal, a sodium inorganic salt as the promoter, and Al₂O₃ as the support.

## Patentansprüche

1. Verfahren zur Herstellung eines gegebenenfalls substituierten Cyclohexanons, umfassend das Reduzieren eines gegebenenfalls substituierten Phenols in Gegenwart eines Katalysators und von Wasserstoff in einem Reaktor, der weitgehend lösungsmittelfrei ist, wodurch das gegebenenfalls substituierte Cyclohexanon erhalten wird; wobei der Katalysator (i) ein katalytisch aktives Übergangsmetall, (ii) einen Träger, der ein Oxid umfasst, und (iii) einen Promotor, der ein anorganisches Metallsalz umfasst, umfasst,
wobei der Promotor in einer Menge zwischen 0,5 bis 2,5 Gew.-% des Katalysators vorliegt und
wobei der Promotor ein Metallsalz aus der Gruppe bestehend aus Li, Na, K, Rb und Cs umfasst und der Promotor vorzugsweise Natriumcarbonat, Kaliumcarbonat oder eine Kombination davon umfasst.

2. Verfahren nach Anspruch 1, wobei es sich bei dem katalytisch aktiven Übergangsmetall um Rh, Ru, Ir, Ni, Pd, Au, Pt oder eine Kombination davon und vorzugsweise Pd handelt.

3. Verfahren nach Anspruch 1 oder 2, wobei sich das Oxid von einem Element aus der Gruppe bestehend aus einem Erdalkalimetall, einem Übergangsmetall, einem Lanthanid und einem Metall der p-Block-Elemente ableitet.

4. Verfahren nach einem der vorhergehenden Ansprüche, wobei es sich bei dem Oxid um SiO₂, MgO, Al₂O₃, TiO₂, CeO₂, ZrO₂ oder eine Kombination davon handelt und das Oxid vorzugsweise Al₂O₃ umfasst.

5. Verfahren nach einem der vorhergehenden Ansprüche, wobei das katalytisch aktive Übergangsmetall in einer Menge zwischen 0,1 und 10 Gew.-% des Katalysators und vorzugsweise zwischen 0,5 und 6 Gew.-% des Katalysators vorliegt.

6. Verfahren nach einem der vorhergehenden Ansprüche, wobei es sich bei dem Katalysator um einen reduzierten Katalysator handelt, der bei einer Durchflussrate von 50 mL/min bis 150 mL/min und bei einer Temperatur von 100 °C bis 400 °C mit Wasserstoffgas behandelt wird.

7. Verfahren nach einem der vorhergehenden Ansprüche, wobei der Katalysator das gegebenenfalls substituierte Phenol unter einem Wasserstoffdruck zwischen 1 bar und 20 bar kontaktiert.

8. Verfahren nach einem der vorhergehenden Ansprüche, wobei der Wasserstoff in der Flüssigphase oder Gasphase, in einem diskontinuierlichen System oder in einem kontinuierlichen Reaktor oder bei einer Temperatur zwischen 80 °C und 250 °C und vorzugsweise zwischen 90 °C und 170 °C mit dem Wasserstoff reagiert.

9. Verfahren nach einem der vorhergehenden Ansprüche, wobei der Katalysator in einer Menge zwischen 0,5 und 10 Gew.-% des gegebenenfalls substituierten Phenols und vorzugsweise zwischen 1 und 5 % vorliegt.

10. Verfahren nach einem der vorhergehenden Ansprüche, wobei das gegebenenfalls substituierte Phenol mit 1 bis 5 Substituenten substituiert ist, bei denen es sich jeweils unabhängig um eine C₁-C₁₂-Alkylgruppe handelt.

11. Verfahren nach einem der vorhergehenden Ansprüche, wobei das substituierte Phenol in der 4-Position mit einem C₁-C₁₂-Alkyl substituiert ist und das C₁-C₁₂-Alkyl aus der Gruppe bestehend aus Methyl, Ethyl, Propyl, Isopropyl, *tert*.-Butyl, Isobutyl, *tert*.-Amyl, Isoamyl, *n*-Hexyl, 1-Methylpentyl und 1,1-Dimethylbutyl ausgewählt ist.

12. Verfahren nach einem der vorhergehenden Ansprüche, wobei es sich bei dem gegebenenfalls substituierten Cyclohexanon um 4-*tert*.-Amylcyclohexanon handelt und es sich bei dem gegebenenfalls substituierten Phenol um 4-*tert*.-Amylphenol handelt.

13. Verfahren nach einem der vorhergehenden Ansprüche, wobei der Katalysator Pd als katalytisch aktives Übergangsmetall, ein anorganisches Natriumsalz als Promotor und Al₂O₃ als Träger umfasst.

## Revendications

1. Procédé de préparation d'une cyclohexanone éventuellement substituée, comprenant la réduction d'un phénol éventuellement substitué en présence d'un catalyseur et d'hydrogène dans un réacteur sensiblement exempt d'un solvant, obtenant ainsi la cyclohexanone éventuellement substituée ;
le catalyseur comprenant (i) un métal de transition catalytiquement actif, (ii) un support comprenant un oxyde, et (iii) un promoteur comprenant un sel métallique inorganique,
le promoteur étant présent en une quantité comprise entre 0,5 et 2,5 % en poids du catalyseur ; et
le promoteur comprenant un sel métallique choisi dans le groupe constitué par Li, Na, K, Rb, et Cs, et préférablement le promoteur comprenant du carbonate de sodium, du carbonate de potassium, ou une combinaison correspondante.

2. Procédé selon la revendication 1, le métal de transition catalytiquement actif étant Rh, Ru, Ir, Ni, Pd, Au, Pt, ou une combinaison correspondante, et préférablement Pd.

3. Procédé selon la revendication 1 ou 2, l'oxyde étant issu d'un élément choisi dans le groupe constitué par un métal alcalino-terreux, un métal de transition, un lanthanide, et un métal élément du bloc p.

4. Procédé selon l'une quelconque des revendications précédentes, l'oxyde étant SiO₂, MgO, Al₂O₃, TiO₂, CeO₂, ZrO₂, ou une combinaison correspondante, et préférablement l'oxyde comprenant Al₂O₃.

5. Procédé selon l'une quelconque des revendications précédentes, le métal de transition catalytiquement actif étant présent en une quantité comprise entre 0,1 % et 10 % en poids du catalyseur, et préférablement entre 0,5 % et 6 % en poids du catalyseur.

6. Procédé selon l'une quelconque des revendications précédentes, le catalyseur étant un catalyseur réduit qui est traité avec un gaz à l'hydrogène à un débit de 50 mL/min à 150 mL/min et à une température de 100 °C à 400 °C.

7. Procédé selon l'une quelconque des revendications précédentes, le catalyseur étant en contact avec le phénol éventuellement substitué sous une pression d'hydrogène comprise entre 1 bar et 20 bars.

8. Procédé selon l'une quelconque des revendications précédentes, l'hydrogène réagissant avec le phénol éventuellement substitué en phase liquide ou en phase gazeuse, dans un système de lot ou dans un réacteur en continu, ou à une température comprise entre 80 °C et 250 °C, et préférablement entre 90 °C et 170 °C.

9. Procédé selon l'une quelconque des revendications précédentes, le catalyseur étant présent en une quantité comprise entre 0,5 % et 10 % en poids du phénol éventuellement substitué, et préférablement entre 1 % et 5 %.

10. Procédé selon l'une quelconque des revendications précédentes, le phénol éventuellement substitué étant substitué par 1 à 5 substituants, chacun desquels étant, indépendamment, un groupe C₁₋₁₂-alkyle.

11. Procédé selon l'une quelconque des revendications précédentes, le phénol éventuellement substitué étant substitué par un C₁₋₁₂-alkyle au niveau de la position 4, et le C₁₋₁₂-alkyle étant choisi dans le groupe constitué par méthyle, éthyle, propyle, *iso*propyle, *tert*-butyle, *iso*butyle, *tert*-amyle, *iso*amyle, *n*-hexyle, 1-méthylpentyle, et 1,1-diméthylbutyle.

12. Procédé selon l'une quelconque des revendications précédentes, la cyclohexanone éventuellement substituée étant la 4-*tert*-amylcyclohexanone, et le phénol éventuellement substitué étant le 4-*tert*-amylphénol.

13. Procédé selon l'une quelconque des revendications précédentes, le catalyseur comprenant du Pd en tant que métal de transition catalytiquement actif, un sel inorganique de sodium en tant que promoteur, et Al₂O₃ en tant que support.
